# EUROPEAN PATENT APPLICATION

(11) **EP 3 895 748 A2**
(43) Date of publication of application: **20.10.2021**
(21) Application number: 20732465.8
(22) Date of filing: 12.02.2020
(51) Int. Cl.: A61M 5/32, A61M 5/31, A61M 5/178

(54) **SAFE INJECTOR NEEDLE**

(30) Priority: 12.12.2018 CN 201811518065
(71) Applicant: Kunshan Medlineer Medical Device Technology Company, Suzhou, Jiangsu 215300 (CN)
(72) Inventor: LI, Gang, Suzhou, Jiangsu 215300 (CN); ZHOU, Yanping, Suzhou, Jiangsu 215300 (CN); BI, Kun, Suzhou, Jiangsu 215300 (CN)
(74) Representative: Kurig, Thomas
(86) International application number: PCT/CN2020/074862
(87) International publication number: WO 2020/119832

(57) **Abstract**

The invention provides a safety injector needle. The safety injector needle includes a needle seat, an injection needle axially fixedly connected to the needle seat, and a trigger protection assembly assembled on the needle seat, the needle seat includes an assembly part and a fixing part; the trigger protection assembly includes a case, a trigger mechanism and an elastic element; the case is arranged in a hollow tube shape; the trigger mechanism includes a first trigger mechanism and a second trigger mechanism. The first trigger mechanism can slide along the extension direction of the case under the action of the elastic element.; the second trigger mechanism can slide along the extension direction of the needle seat under the action of the elastic element, so that the injection needle is completely contained between the trigger mechanism and the needle seat after the safety injector needle is used. The safety injector needle of the present invention not only has better protection function, but also has the function of indicating the state of use, and has a simple structure, low production cost, and is suitable for popularization and use.

## Description

This application claims the priority of the Chinese patent application whose application date is December 12, 2018, the application number is 201811518065.X, and the invention name is "Safety Injector Needle", the entire content of which is incorporated into this application by reference.

### BACKGROUND

### 1. TECHNICAL FIELD

The present invention relates to a safety injector needle, which belongs to the field of medical equipment.

### 2. DESCRIPTION OF RELATED ART

A syringe is a common medical appliance, which usually includes an injection syringe, a needle connected to the injection syringe, and a protective sleeve covering the needle; in the process of use, firstly remove the protective sleeve and expose the needle to complete the injection; Separate the needle and the syringe barrel after use and discard them separately. On the one hand, avoid multiple uses of the syringe/needle, and on the other hand, prevent cross-infection caused by accidental contact with the used needle.

However, in the process of using the syringe, the exposed needle tip will bring a great psychological burden to the patient, and it is easy to cause harm to the human body due to accidental contact with the needle, which reduces the user friendliness of the syringe in the prior art. Furthermore, in the actual recycling process of discarded syringes/needles, due to the lack of a protective sleeve, the needles are likely to pierce the containment package, causing damage to the human body, and it is impossible to effectively avoid cross-infection caused by accidentally touching the needles; at the same time, in the prior art, there is no significant difference between the needle after use and the needle before use, and it is impossible to judge whether the needle is used or not based on the appearance of the needle, which may easily lead to the repeated use of discarded needles.

In view of this, it is indeed necessary to provide a new safety injector needle to solve the above-mentioned problems.

### SUMMARY

The purpose of the present invention is to provide a safety injector needle to mark the use state of the safety injector needle while ensuring the safety of the safety injector needle.

In order to achieve the above-mentioned object of the invention, the present invention provides a safety injector needle, including a needle seat, an injection needle axially fixedly connected to the needle seat, and a trigger protection assembly assembled on the needle seat;

The needle seat includes an assembly part and a fixing part, and the assembly part is arranged in a hollow tube shape;

The trigger protection assembly includes a case, a trigger mechanism and an elastic element;

The case is arranged in a hollow tube shape and is fixedly connected to the assembling part, and the inner wall of the case is provided with a first displacement chute, a second displacement chute and a locking chute corresponding to the trigger mechanism.

The trigger mechanism includes a first trigger mechanism and a second trigger mechanism. The first trigger mechanism is provided with a first inclined guide surface, a limiting slider corresponding to the first displacement chute and the second displacement chute, and an elastic slider corresponding to the locking chute; the second trigger mechanism is provided with a second inclined guide surface for driving the first trigger mechanism to rotate in the axial direction of the assembling part;

Under the action of external force, the first inclined guide surface and the second inclined guide surface cooperate with each other to drive the limiting slider and the elastic slider to rotate in the axial direction of the assembling part, so that the limiting slider turns from the first displacement chute to the second displacement chute, and when the limiting slider slides into the second displacement chute, the elastic slider is clamped in the locking chute inside.

As a further improvement of the present invention, the first displacement chute and the second displacement chute are arranged at intervals by a baffle, and the bottom of the baffle is provided with a through groove for the limiting slider to pass through, so a partition board is provided between the second displacement chute and the locking chute to limit the rotation direction and the rotation angle of the first trigger mechanism in the case.

As a further improvement of the present invention, a lug boss is provided at the bottom of the locking chute, and the elastic slider is clamped on the lug boss to limit the locking position of the first trigger mechanism.

As a further improvement of the present invention, the first trigger mechanism includes an extension portion and an accommodating portion, the extension portion protrudes from the case and partially receives the fixing part and/or the injection needle; the accommodating portion extends in the opposite direction from the extension portion and is accommodated in the case. The accommodating portion includes a first guide wall, and the second trigger mechanism partially penetrates through the assembling part and can slide along the axial direction of the assembling part trigger mechanism assembly department assembly department

As a further improvement of the present invention, the first inclined guide surface is provided on a side surface of the first guide wall extending in the axial direction axial direction of the assembling part, and the limiting slider and the elastic slider are arranged on the outer wall of the first guide wall.

As a further improvement of the present invention, the first guide wall is provided with at least two accommodating cavities for accommodating the elastic element, the accommodating cavity is provided with a first resisting wall, the elastic element is held between the first resisting wall and the second trigger mechanism to elastically connect the second trigger mechanism and the first trigger mechanism.

As a further improvement of the present invention, the second trigger mechanism includes a second guide wall provided corresponding to the first guide wall and an elastic arm provided at a distance from the second guide wall, and the second guide wall and the elastic arm all penetrate the assembling part.

As a further improvement of the present invention, the second inclined guide surface is provided on a side surface of the second guide wall extending in the axial direction of the assembling part, and corresponds to the first inclined guide surface, and the assembling part. A through hole for accommodating the second guide wall and the elastic arm is provided, and the through hole wall of the through hole close to the second inclined guide surface is inclined. When the axial direction of the assembling part moves toward the first trigger mechanism, the through hole wall abuts on the second inclined guide surface to push the second trigger mechanism to rotate in the axial direction of the assembling part.

As a further improvement of the present invention, a first positioning block is provided on the second guide wall, a second positioning block and a third positioning block are provided on the elastic arm, and the direction of the fixing part is defined as the positive direction, so the first positioning block is located above the third positioning block, and the second positioning block is located above the third positioning block.

As a further improvement of the present invention, the assembling part is provided with a first groove for accommodating the first/third positioning block and a second groove for accommodating the third positioning block, defining the direction where the fixing part is located is the positive direction, the first groove is located above the second groove, and when the first positioning block is clamped in the first groove, the third positioning block is clamped in the second groove, the second positioning block enters the assembling part and is held against the inner side of the top wall of the assembling part, and the injection needle is accommodated in the second trigger mechanism.

The advantages and positive effects of the present invention are introduced as follows:

The safety injector needle of the present invention is provided with a case, a trigger mechanism and an elastic element, the trigger mechanism can slide along the extension direction of the case under the action of the elastic element, so that all the injection needles are contained between the trigger mechanism and the needle seat after the safety injector needle is used, the safety of use is ensured; at the same time, the safety injector needle of the present invention has a simple structure, better safety in use, and low production cost, which is suitable for popularization and use.

### BRIEF DESCRIPTION OF THE DRAWINGS

FIG. 1 is an exploded view of the safety injector needle and the main body of the shield in the first embodiment of the present invention.
FIG. 2 is a schematic diagram of the structure of the injection assembly in FIG. 1.
FIG. 3 is a schematic structural view of the needle seat in FIG. 2 from another angle.
FIG. 4 is a cross-sectional view of the needle seat in FIG. 2.
FIG. 5 is an exploded view of the trigger mechanism in FIG. 1.
FIG. 6 is a schematic diagram of the combination of the trigger mechanism and the injection assembly in FIG. 1.
FIG. 7 is a schematic diagram of the structure of the case in FIG. 1.
FIG. 8 is a cross-sectional view of the case in FIG. 7.
FIG. 9 is a schematic diagram of the structure of the first trigger mechanism in FIG. 6.
FIG. 10 is a schematic diagram of the structure of the first trigger mechanism shown in FIG. 9 from another angle.
FIG. 11 is a schematic diagram of the structure of the second trigger mechanism in FIG. 6.
FIG. 12 is a schematic diagram of the structure of the second trigger mechanism and the injection assembly in FIG. 6 after being combined.
FIG. 13 is a cross-sectional view of the safety injector needle at different positions.
FIG. 14 is an exploded view of the safety injector needle in the second embodiment of the present invention.
FIG. 15 is a schematic diagram of the structure of the case in FIG. 14.
FIG. 16 is a schematic diagram of the structure of the first trigger mechanism in FIG. 14.
FIG. 17 is a schematic diagram of the structure of the first trigger mechanism shown in FIG. 16 from another angle.
FIG. 18 is an exploded view of the safety injector needle in the third embodiment of the present invention.
FIG. 19 is a schematic diagram of the structure of the needle seat in FIG. 18.
FIG. 20 is a schematic structural view of the needle seat shown in FIG. 19 from another angle.
FIG. 21 is a schematic diagram of the structure of the second trigger mechanism in FIG. 18.
FIG. 22 is a schematic diagram of the combination of the second trigger mechanism and the needle seat in FIG. 18.

### DETAILED DESCRIPTION OF THE ILLUSTRATED EMBODIMENT

In order to make the objectives, technical solutions and advantages of the present invention clearer, the present invention will be described in detail below with reference to the accompanying drawings and specific embodiments.

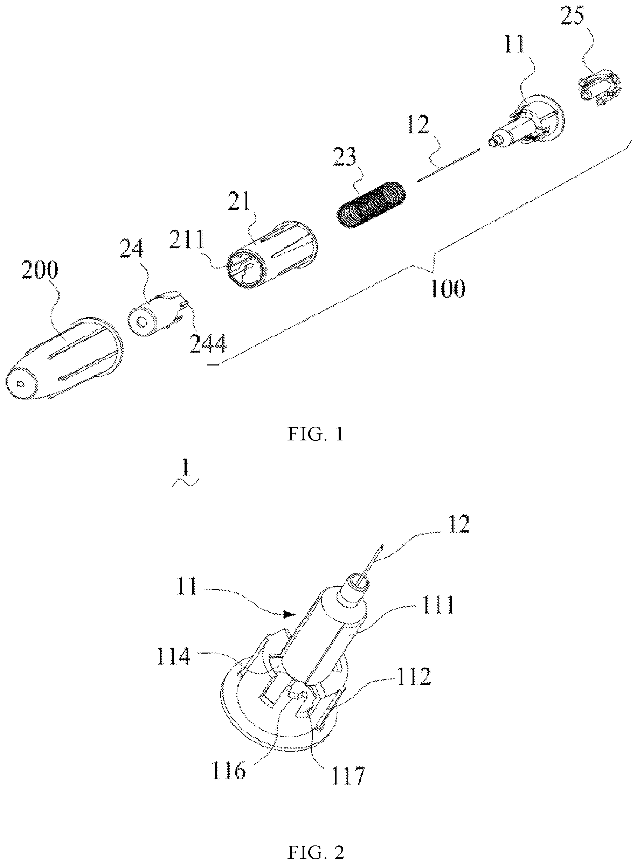

Please refer to FIG. 1, FIG. 2 and FIG. 5, which are the safety injector needle 100 in the first embodiment of the present invention. The safety injector needle 100 includes an injection assembly 1 and a trigger protection assembly 2 assembled on the injection assembly 1. The trigger protection assembly 2 is arranged outside the injection assembly 1 to prevent the injection assembly 1 from causing harm to the human body after use.

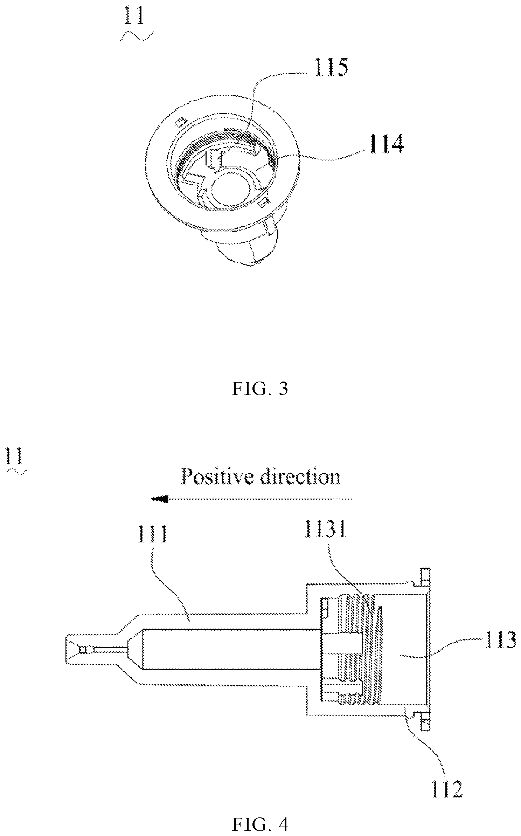

Please refer to FIGS. 2 to 4, the injection assembly 1 includes a needle seat 11 and an injection needle 12 axially fixedly connected with the needle seat 11. In the present invention, the needle seat11 includes a fixing part 111 and an assembling part 112, and the axes of the fixing part 111 and the assembling part 112 are located on the same straight line; specifically, the fixing part 111 is provided on the assembling part 112 and along the assembling part 112. The axis of 112 extends outward; the assembling part 112 is provided with a cylindrical inner cavity 113, the width/diameter of the fixing part 111 is smaller than the diameter of the inner cavity 113, and in this embodiment, the inner wall of the inner cavity 113 is provided for clamped connecting the connection portion 1131 of the injection device (not shown); preferably, the connection portion 1131 is threaded. Of course, in other embodiments, the connection portion 1131 can also be provided in other forms, as long as the safety injector needle 100 is firmly connected to the injection device.

Further, it is defined that the end of the injection needle 12 away from the assembling part 112 is the distal end, and the end close to the assembling part 112 is the proximal end, and the proximal end of the injection needle 12 penetrates the fixing part 111 and the assembling part 112 and is accommodated in the inner cavity 113; the distal end extends along the axial direction of the fixing part 111 toward a side away from the assembling part 112 and is fixedly connected to the fixing part 111 to prevent the injection needle 12 from sliding along the axial direction of the needle seat 11.

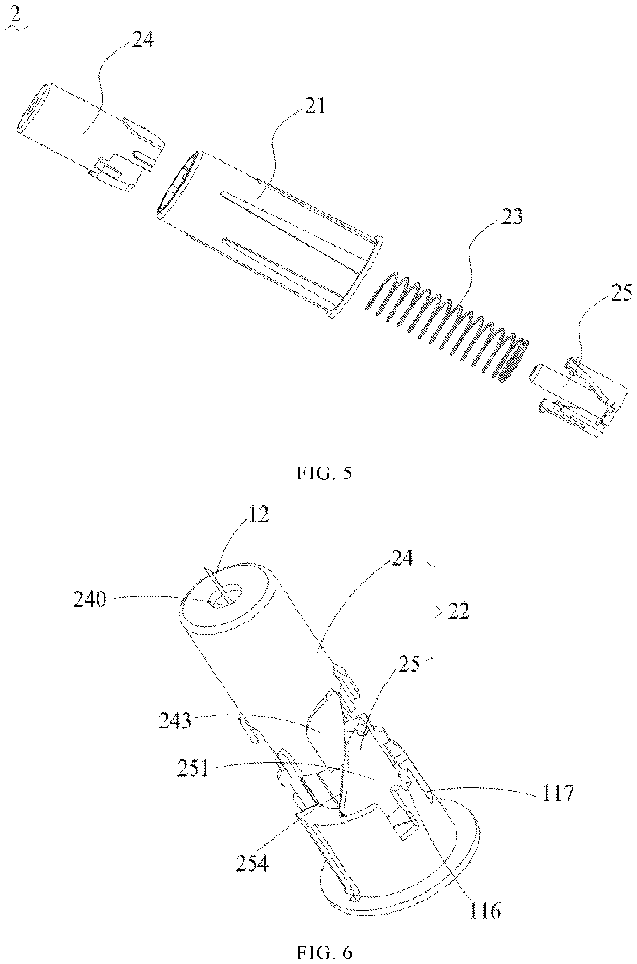

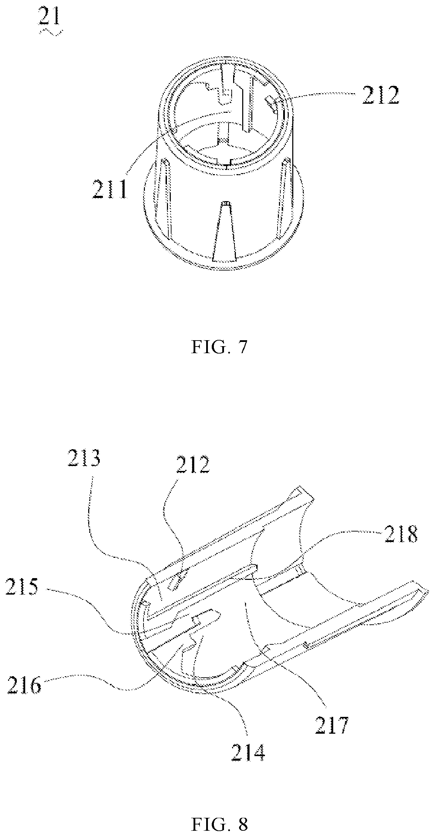

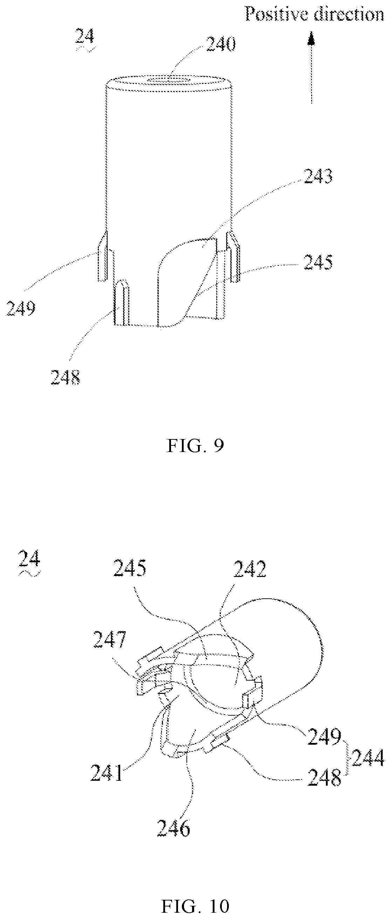

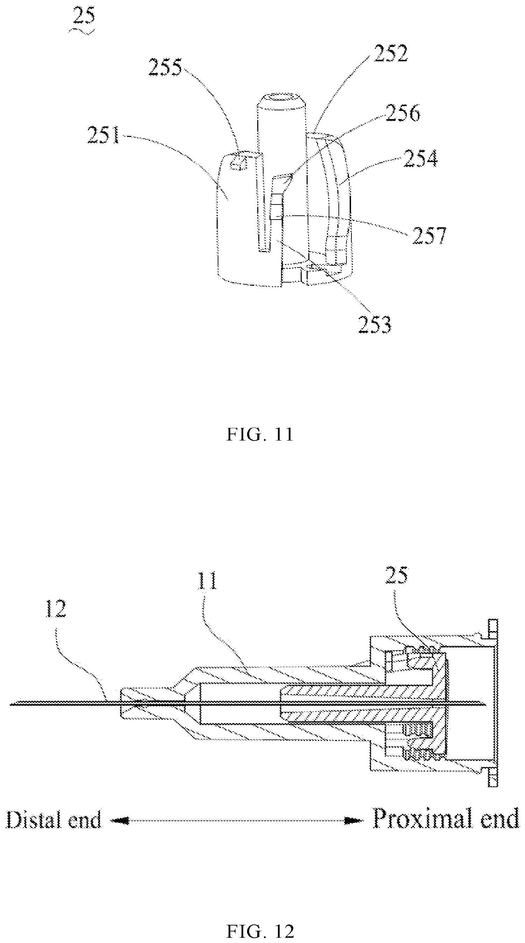

Please refer to FIGS. 5 to 12 in combination with FIG. 1, the trigger protection assembly 2 includes a case 21, a trigger mechanism 22 and an elastic element 23. The case 21 is arranged on the needle seat 11. In the present invention, the case 21 has a hollow tubular shape. The side of the needle seat 11 close to the assembly part 112 is defined as the inside, and the side close to the fixing part 111 is defined as the outside. One end of the case 21 is clamped and connected to the assembling part 112, the other end extends outward along the axial direction of the needle seat 11 and contains the fixing part 111, a containment space is formed for containing the trigger mechanism 22 and the elastic element 23. Preferably, in the present invention, the case 21 and the needle seat 11 are detachably connected. Of course, in other embodiments, the case 21 and the needle seat 11 may also be integrally provided, that is, in the present invention. The connection method between the case 21 and the needle seat 11 can be selected according to actual needs, and is not limited here.

The trigger component 22 includes a first trigger mechanism 24 arranged in a hollow and sleeved outside the fixing part 111 and a second trigger mechanism 25 provided corresponding to the first trigger mechanism 24. The first trigger mechanism 24 includes an accommodating portion 241 and an extension portion 242; the accommodating portion 241 is received in the case 21 and slidably connected to the case 21, and the accommodating portion 241 includes a first guide wall 243 and slider 244 arranged on the outer wall of the accommodating portion 241.

On the one hand, the first guide wall 243 can be used to define the connection position of the first trigger mechanism 24 and the case 21; on the other hand, it can guide the first trigger mechanism 24 to rotate in the axial direction of the needle seat 11 under the action of external force, specifically, the first guide wall 243 is provided with an inclined first inclined guide surface 245, and the first inclined guide surface 245 is arranged on a side surface of the first guide wall 243 extending in the axial direction of the assembling part 112, in this embodiment, the first guide wall 243 is provided with at least two accommodating cavities 246 for accommodating the elastic element 23, the accommodating cavity 246 is provided with a first resisting wall 247, the elastic element 23 resists between the first resisting wall 247 and the second trigger mechanism 25 to elastically connect to the first trigger mechanism 24 and the second trigger mechanism 25. Of course, in other embodiments, the first guide wall 243 can also be provided with one, that is, in the present invention, the number of the first guide wall 243 can be selected according to needs, which is not limited here.

The slider 244 is used to define the sliding position and sliding direction of the trigger mechanism 22, and the inner wall of the case 21 is provided with a chute 211 corresponding to the slider 244, and the bottom end of the chute 211 is provided with a position for positioning the lug boss 212 of the slider 244. In this embodiment, the slider 244 includes a limiting slider 248 and a locking slider 249 that is spaced apart from the limiting slider 248, wherein the limiting slider 248 is used to limit the sliding position of the first trigger mechanism 24 and in the sliding direction, the locking slider 249 is used to define the locking position of the first trigger mechanism 24 and the case 21, and in this embodiment, the locking slider 249 is an elastic slider that can be elastically deformed, such as an elastic piece.

The chute 211 includes a displacement chute corresponding to the limit slider 248 and a locking chute 213 corresponding to the locking slider 249. The displacement chute includes a first displacement chute 214 and a second displacement chute 215 that is spaced apart from the first displacement chute 214. The connection position of the fixing part 111 and the assembling part 112 is defined as the origin, and the extension direction of the fixing part 111 is in the positive direction, in the positive direction, the chute 211 has a top end far away from the origin and a bottom end close to the origin. In this embodiment, the first displacement chute 214 and the second displacement chute 215 are spaced apart by the baffle 216, and block the bottom end of the baffle 216 is provided with a through groove 217 through which the limiting slider 248 passes, so that the bottom end of the first displacement chute 214 and the bottom end of the second displacement chute 215 are connected to each other.

The locking chute 213 and the displacement chute are spaced apart. In this embodiment, a partition 218 is provided between the locking chute 213 and the second displacement chute 215. When the first trigger mechanism 24 slides in the case 21, the first guide wall 243 at least partially interferes with the partition 218 to limit the rotation direction and rotation angle of the first trigger mechanism 24 in the case 21. Further, the lug boss 212 is provided at the bottom of the locking slide groove 213 to position the locking slider 249.

The extension portion 242 extends from the accommodating portion 241 in the axial direction (namely, the positive direction) of the needle seat 11, the extension portion 242 protrudes from the case 21 and partially accommodates the fixing part 111 and the injection needle 12. A via 240 for receiving the injection needle 12 is provided.

In this embodiment, the first trigger mechanism 24 can slide from the initial position in the case 21 to the locked position through the injection position. Specifically, when the first trigger mechanism 24 is in the initial position, the first guide wall 243 is locked. Hold inside the case 21, the limit slider 248 is received at the top end of the first displacement chute 214, the injection needle 12 is completely received in the extension portion 242, and the accommodating part 241 is completely received in the case 21; when a trigger mechanism 24 slides from the initial position to the injection position, the first trigger mechanism 24 moves along the top end of the first displacement chute 214 to the bottom end of the first displacement chute 214.

When the first trigger mechanism 24 slides to the injection position, the limiting slider 248 slides to the through groove 217, the accommodating portion 241 and the extending portion 242 are completely contained in the case 21, and the injection needle 12 passes through the through hole 240, and then acts on the patient to complete drug injection; after the drug injection is completed, the first trigger mechanism 24 slides from the injection position to the locked position, and the limiting slider 248 slides out of the through groove 217, enters the second displacement chute 215, and slides from the bottom end of the second displacement chute 215 to the top end, at this time, the locking slider 249 enters the locking chute 213 and moves to the top of the case 21 with the first trigger mechanism 24.

When the first trigger mechanism 24 slides to the locking position, the first guide wall 243 is clamped on the top of the case 21, the limit slider 248 is received at the top of the second displacement chute 215, and the locking slider 249 slides to lock the chute 213, and one end of the locking slider 249 abuts against the top end of the locking chute 213, and the other end is clamped on the lug boss 212 to lock the position of the first trigger mechanism 24. At this time, the extension portion 242 and at least part of the accommodating portion 241 protrude outward from the top end of the case 21, and the elastic element 23 is exposed to the outside of the case 21 through the accommodating portion 241 to indicate that the safety injector needle 100 has been used; further, injection the needle 12 is again housed in the extension portion 242 to prevent the used injection needle 12 from causing harm to the human body and effectively avoid cross-infection caused by accidentally touching the used injection needle 12.

The second trigger mechanism 25 partially penetrates the assembling part 112 and can move along the axial and/or axial direction of the assembly department 112. The second trigger mechanism 25 includes a second guide wall 251, a second resisting wall 252, and an elastic arm 253. In this embodiment, the second guide wall 251, the second resisting wall 252, and the elastic arm 253 all penetrate the assembly part 112, and the injection needle 12 penetrates the second trigger mechanism 25 along the axial direction of the needle seat 11, and can slide relatively with the second trigger mechanism 25.

The second guide wall 251 is disposed corresponding to the first guide wall 243, and the second guide wall 251 is provided with a second inclined guide surface 254 corresponding to the first inclined guide surface 245, specifically, the second inclined guide surface 254 is provided on a side surface of the second guide wall 251 that extends along the axial direction of the assembling part 112; the second resisting wall 252 is provided on the top end of the second guide wall 251 and is formed along the axial direction of the assembling part 112, and the second resisting wall 252 is arranged corresponding to the first resisting wall 247, the elastic element 23 is clamped between the first resisting wall 247 and the second resisting wall 252, and when the elastic element 23 is compressed, the first guiding wall 243 interferes with the second guide wall 251, and the first inclined guide surface 245 abuts against the second inclined guide surface 254, so that the first trigger mechanism 24 rotates along the axial direction of the assembling part 112 under the guidance of the second trigger mechanism 25..

The elastic arm 253 is spaced apart from the second guide wall 251, and the elastic arm 253 is arranged on a side of the second guide wall 251 away from the second inclined guide surface 254.

Further, the assembling part 112 is provided with a through hole 114 for receiving the second guide wall 251 and the elastic arm 253, and the through hole wall 115 of the through hole 114 close to the second inclined guide surface 254 is arranged obliquely, when the second trigger mechanism 25 moves toward the first trigger mechanism 24 along the axial direction of the assembling part 112, the through hole wall 115 abuts on the second inclined guide surface 254 to push the second trigger mechanism 25 to rotate in the axial direction of the assembling part 112.

Both the second guide wall 251 and the elastic arm 253 are provided with positioning blocks. In this embodiment, the positioning blocks include a first positioning block 255 arranged on the second guide wall 251, a second positioning block 256 and the third positioning block 257 arranged on the elastic arm 253. In the positive direction, the first positioning block 255 is located above the third positioning block 257, and the second positioning block 256 is located above the third positioning block 257.

The assembling part 112 is provided with a groove corresponding to the positioning block. The groove includes a first groove 116 for receiving the first positioning block 255/the third positioning block 257 and a groove 117 for receiving the third positioning block 257. In the positive direction, the first groove 116 is located above the second groove 117.

In the present invention, the second trigger mechanism 25 can be slid from the initial position to the locked position in the assembling part 112 via the ready position. Specifically, when the second trigger mechanism 25 is in the initial position, the third positioning block 257 is clamped in the first groove 116, and at this time, the second guide wall 251 and the elastic arm 253 partially penetrate the assembling part 112 and are accommodated in the between the case 21 and the fixing portion 111, the proximal end part/all of the injection needle 12 is contained in the second trigger mechanism 25. This arrangement can effectively prevent the injection needle 12 from being contaminated by the external environment before use; on the other hand, this arrangement can effectively prevent the injection needle 12 from being contaminated by the external environment before use. The axial position of the injection needle 12 can be effectively positioned, and when the injection device is connected to the inner cavity 113, the injection needle 12 can be prevented from shifting or bending.

Further, the injection device is connected to the needle seat11 through the connecting portion 1131. In this embodiment, the injection device will push the second trigger mechanism 25 along the needle seat under the action of external force during the process of connecting the injection device to the assembling part 112 through threads. The axis of 11 slides from the initial position to the ready position. During the sliding process, the through hole wall 115 abuts on the second inclined guide surface 254, pushing the second trigger mechanism 25 to rotate in the axial direction of the assembling part 112 until the second guide wall 251 and the elastic arm 253 all penetrate the through hole 114 and reach the ready position of the second trigger mechanism 25. At this time, the first trigger mechanism 24 is still in the initial position, the elastic element 23 is compressed to abut against the second resisting wall 252, and the proximal end of the injection needle 12 penetrates the second trigger mechanism 25, and plugged into the injection device.

After the drug injection is finished, when the injection device is removed, the elastic element 23 drives the second trigger mechanism 25 to slide along the axial direction of the needle seat 11 toward the side away from the fixing portion 111 under its own elastic force, until it slides to the locked position when the second trigger mechanism 25 is in the locked position, the first positioning block 255 is clamped in the first groove 116, the third positioning block 257 is clamped in the second groove 117, and the second positioning block 256 under the action of the elastic arm 253, it enters into the assembling part 112 and abuts against the inner side of the top wall of the assembling part 112. At this time, the top end of the injection needle 12 is partially/all contained in the second trigger mechanism 25. Preferably, in this embodiment, when the second trigger mechanism 25 is in the locked position, all the injection needles 12 are housed in the second trigger mechanism 25 to prevent the injection needle 12 from causing harm to the patient after use.

The elastic element 23 is elastically connected to the trigger mechanism 22 and the needle seat 11, so that when the trigger mechanism 22 is pressed, the trigger mechanism 22 is displaced between the initial position and the locked position. In this embodiment, the elastic element 23 is accommodated in the case. The accommodating portion 241 is transparently arranged. At the position where the first abutting wall 247 abuts the elastic element 23, the elastic element 23 is arranged close to the inner wall of the accommodating portion 241. When the first trigger mechanism 24 is in the initial position, the accommodating portion 241 is contained in the case 21, the elastic element 23 cannot be observed through the accommodating portion 241; when the first trigger mechanism 24 is in the locked position, part of the accommodating portion 241 protrudes from the top end of the case 21, so that at least part of the elastic element 23 protrudes from the case 21 through the accommodating portion 241 to be observed..

Of course, in other embodiments, the accommodating portion 241 may also be provided with an observation hole, so that the elastic element 23 can be observed through the observation hole, that is, in the embodiment of the present invention, the specific arrangement form of the accommodating portion 241 may be selected according to actual needs, and it is only necessary to ensure that the elastic element 23 can be observed through the accommodating portion 241 at the locked position.

In the present invention, the elastic element 23 is a spring, and when the first trigger mechanism 24 is in the locked position, at least part of the spring (including the half-turn spring, one-turn spring or multiple-turn springs) is exposed to the outside of the case 21 through the accommodating portion 241. Of course, in another embodiment of the present invention, the elastic element 23 can also be a combination of an indicating member (not shown) and an elastic member (not shown). The indicating member and the elastic member are housed in the accommodating portion 241 to indicate the member is arranged close to the inner wall of the accommodating portion 241, and the elastic member abuts on the first resisting wall 247 through the indicating member. When the first trigger mechanism 24 is in the locked position, the elastic member continues to be accommodated in the accommodating portion 241, and the indicating member is exposed to the outside of the case 21 through the accommodating portion 241.

Further, the elastic element 23 may also be provided with an identification mark, which is used to expose the safety injector needle 100 to the outside of the case 21 after use, so as to facilitate the patient/user to quickly and accurately use the safety injector needle 100 for judgment, preferably, in the present invention, the identification mark may be one or more of color mark, texture mark, symbol mark or graphic mark.

It should be noted that in the present invention, the elastic element 23 is only used as a spring or a combination of an indicating member and an elastic member for illustration, but it should not be limited to this. In other embodiments, the elastic element 23 may also be other a separated elastic member or a combination of elastic members and other indicating members that can be elastically connected to the trigger mechanism 22 and the needle seat 11 and displayed through the accommodating portion 241, that is, in the present invention, the specific arrangement form of the elastic element 23 is not limited.

As shown in FIG. 1, the safety injector needle 100 is also connected with a shield for accommodating the safety injector needle 100. The shield includes a shield body 200 and a protective film (not shown) encapsulated at the opening position of the shield body 200, the shield body 200 is sleeved on the outside of the case 21, and the protective film encapsulates the injection assembly 1 and the trigger protection assembly 2 in the shield body 200 to prevent the injection assembly 1 and the trigger protection assembly 2 from being exposed to the external environment and ensure injection assembly 1 and trigger protection assembly 2 is hygienic.

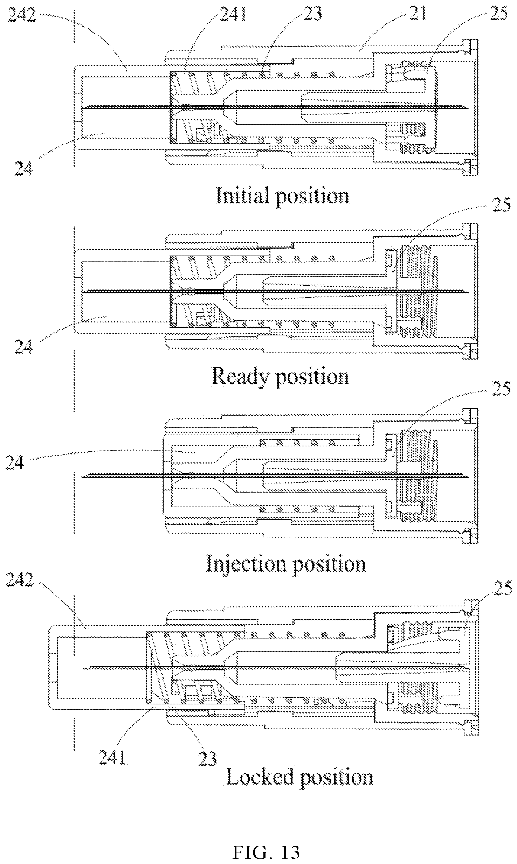

Please refer to FIG. 13, when using the safety injector needle 100 in this embodiment, first tear off the protective film encapsulated on the shield body 200, and then connect the injection device to the inner cavity 113 of the assembling part 112, and inject when the device is connected with the assembling part 112, the second trigger mechanism 25 will be pushed to move along the axial direction of the needle seat 11 in the direction where the fixing part 111 is located, and along the axial direction of the assembling part 112 under the action of the through-hole wall 115 after rotating, when the injection device is connected to the assembling part 112 stably, the second trigger mechanism 25 slides from the initial position to the ready position, and the elastic element 23 is compressed.

Then, the main body 200 of the shield covering the outside of the case 21 is pulled off to expose the safety injector needle 100. At this time, the distal end of the injection needle 12 is housed in the first trigger mechanism 24, and the safety injector needle 100 is transferred to the place where injection is required. When the first trigger mechanism 24 is pressed, the extension portion 242 protruding outside the case 21 slides along the axial direction of the needle seat 11 toward the direction of the assembling part 112 under the action of external force, and slides on the first trigger mechanism 24 In the process, the first inclined guide surface 245 abuts on the second inclined guide surface 254, so that the first trigger mechanism 24 is pushed by the second inclined guide surface 254 to rotate along the axial direction of the assembling part 112, and the slider 248 is restricted. From the first displacement chute 214 into the through groove 217, the elastic element 23 is further compressed, and the injection needle 12 passes through the through hole 240 at the top of the first trigger mechanism 24 to act on the patient, and complete the medicine through the injection device Injection.

After the drug injection is completed, the safety injector needle 100 is removed from the patient. At this time, under the action of the elastic element 23, the limiting slider 248 escapes from the through groove 217 and enters the second displacement chute 215, and moves along the needle seat 11. The shaft slides away from the assembling part 112, so that the extension portion 242 and the partial accommodating portion 241 protrude from the top end of the case 21, and under the cooperation of the locking slider 249 and the locking chute 213 , the first trigger mechanism 24 is locked in the locked position, and at the same time the distal end of the injection needle 12 is housed in the first trigger mechanism 24 again, and at least part of the elastic element 23 can be observed through the accommodating portion 241; further, remove the safety injector needle 100 from the injection device. During the removal process, under the action of the elastic element 23, the second trigger mechanism 25 slides along the axial direction of the needle seat 11 toward the side away from the fixing portion 111, and passes through the second positioning the block 256 is clamped in the assembling part 112, so that the second trigger mechanism 25 is locked in the locked position, and the proximal end of the injection needle 12 is all housed in the second trigger mechanism 25, completing the separation of the safety injector needle 100 and the injection device.

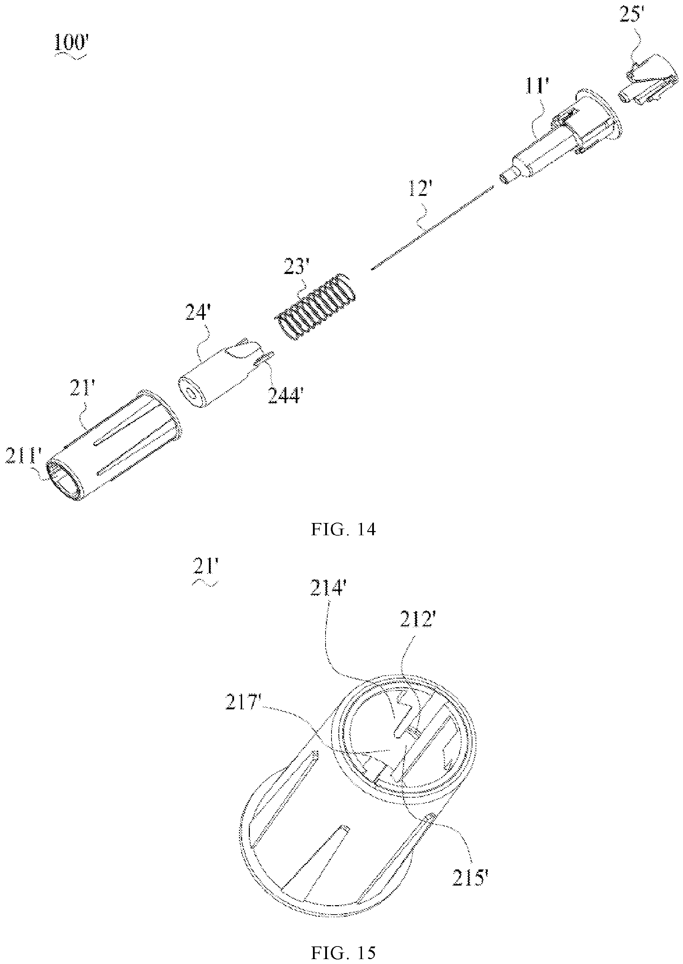

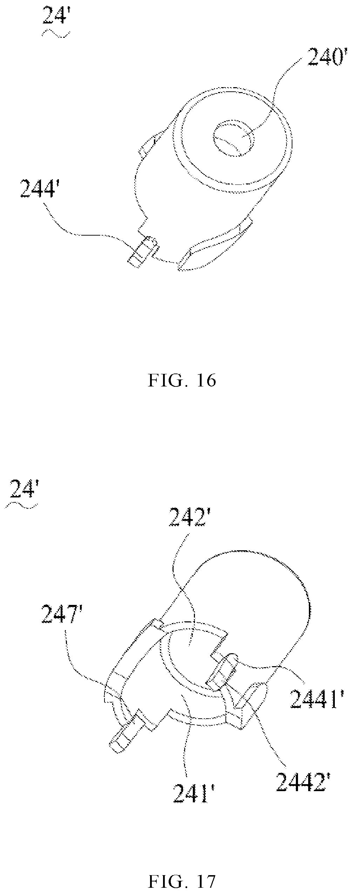

Please also refer to FIGS. 14 to 17, which are the safety injector needle 100' in the second embodiment of the present invention. In this embodiment, the safety injector needle 100' and the safety injector needle 100 in the first embodiment have with substantially the same structure, the safety injector needle 100'includes an injection assembly 1 and a trigger protection assembly 2, the injection assembly 1 includes a needle seat 11' and an injection needle 12', and the trigger protection assembly 2 includes a case 21' and a trigger mechanism 22. The difference lies in the different setting forms of the slider 244' in the first trigger mechanism 24' and the chute 211' in the case 21'. The following description will focus on the difference between the safety injector needle 100' and the safety injector needle 100. For description, the same parts will not be repeated here.

In this embodiment, the slider 244' is a positioning slider used to limit the sliding position and the sliding direction of the trigger mechanism 22, and the slider 244' includes a limiting portion 2441' fixed on the outer wall of the accommodating portion 241' and a locking position 2442' extending beyond the accommodating position 241' from the limiting position 2441' along the axial direction of the needle seat 11'. Preferably, in this embodiment, the locking portion 2442' is provided in the shape of an elastic sheet.

The inner wall of the case 21' is provided with a chute 211' corresponding to the slider 244'. The chute 211' includes a first displacement chute 214' and a second displacement chute 214' spaced apart from the first displacement chute 214'. The bottom end of the second displacement chute 215' is provided with a lug boss 212' for positioning the slider 244'.

In this embodiment, when the first trigger mechanism 24' is in the initial position, the slider 244' is received at the top of the first displacement chute 214'; when the first trigger mechanism 24' slides from the initial position to the injection position, the first trigger mechanism 24' slides from the top end to the bottom end of the first displacement chute 214'; when the first trigger mechanism 24' slides to the injection position, the slider 244' slides to the through groove 217', and the injection needle 12' passes through the through hole 240' to complete the injection of the medicine. After the injection is completed, the slider 244' slides out of the through groove 217', enters the second displacement chute 215', and slides from the bottom to the top of the second displacement chute 215'.

When the first trigger mechanism 24' slides to the locked position, the limiting portion 2441' is received at the top end of the second displacement chute 215', and the locking portion 2442' is clamped on the lug boss 212' to lock the first the position of the trigger mechanism 24'. At this time, the elastic element 23' is exposed to the outside of the case 21' through the accommodating portion 241' to indicate that the safety injector needle 100' has been used; the injection needle 12' is again housed in the extension portion 242' in order to prevent the injection needle 12' from causing harm to the human body after use.

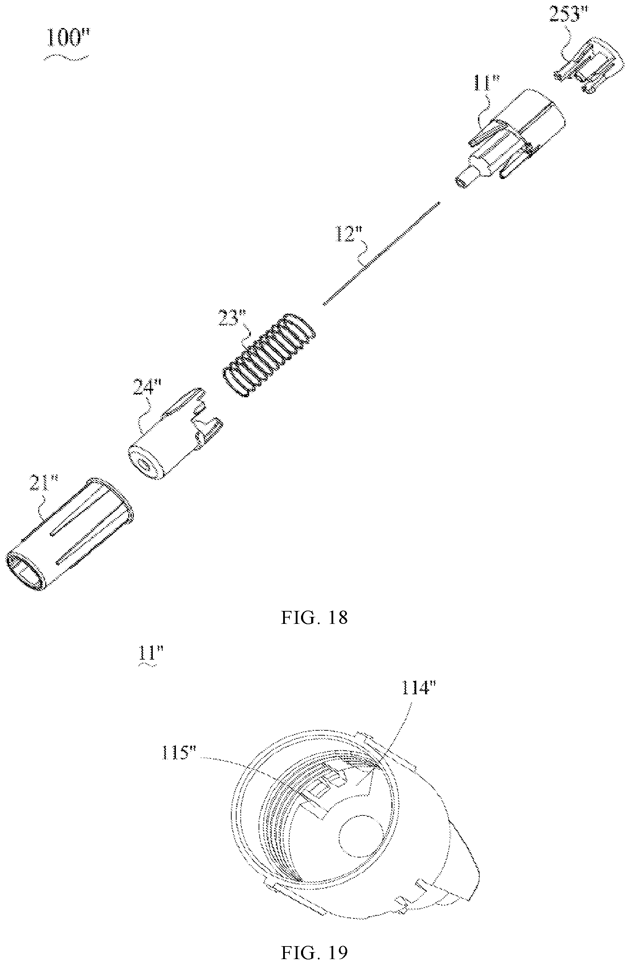

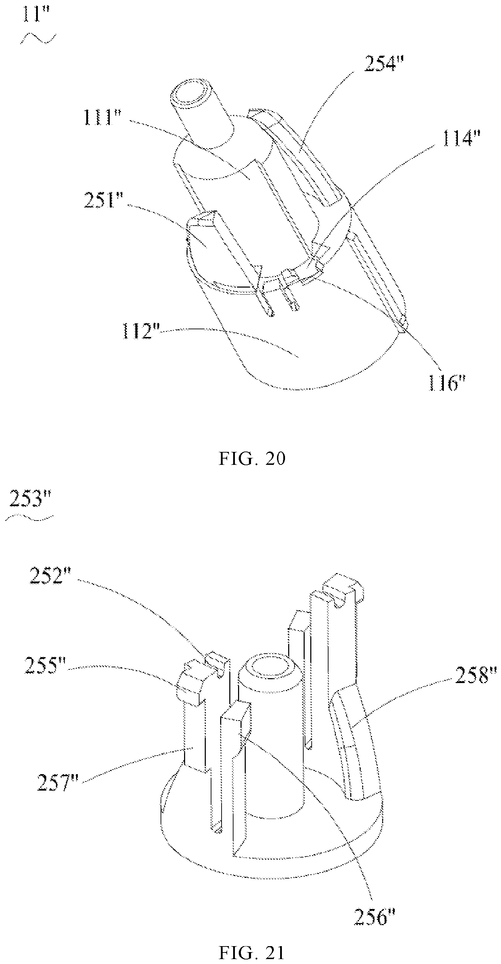

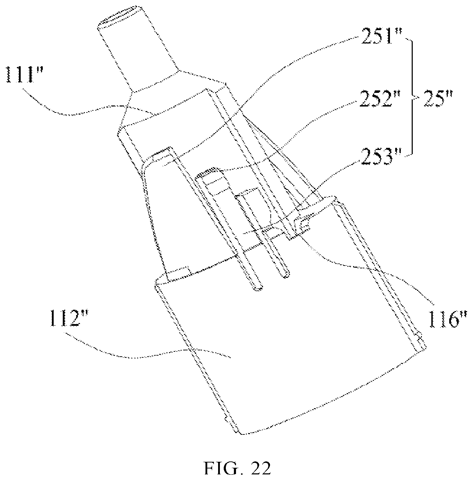

Please also refer to FIGS. 18 to 22, which are the safety injector needle 100' in the third embodiment of the present invention. In this embodiment, the safety injector needle 100' is the same as the safety injector needle in the first embodiment. 100 and the safety injector needle 100' in the second embodiment have substantially the same structure. The safety injector needle 100' includes an injection assembly 1 and a trigger protection assembly 2, and the injection assembly 1 includes a needle seat 11' and an injection needle 12'. The trigger protection assembly 2 includes a case 21" and a trigger mechanism 22. The difference lies in: the setting form of the second trigger mechanism 25" and the connection form between the second trigger mechanism 25" and the needle seat 11" are different. The following description will describe the differences between the safety injector needle 100" and the safety injector needle 100 and 100', and the same parts will not be repeated.

In this embodiment, the second trigger mechanism 25" includes a second guide wall 251", a second resisting wall 252", and an elastic arm 253", and the second guide wall 251" is disposed on the assembling part 112" is formed integrally with the assembling part 112"; further, the assembling part 112" is provided with a through hole 114", and the second resisting wall 252" and the elastic arm 253" passes through the through hole 114"The second resisting wall 252" is provided on the top of the elastic arm 253" and extends along the axial direction of the assembling part 112". The elastic arm 253" is provided on the second guide wall 251" away from the side of the second inclined guide surface 254", and the second resisting wall 252" and the elastic arm 253" can slide relative to the assembling part 112".

The elastic arm 253" is provided with a third inclined guide surface 258" in the axial direction of one side close to the second guide wall 251", and a positioning block is provided on the elastic arm 253". The positioning block includes a first positioning block 255", a second positioning block 256", and a third positioning block 257". The second positioning block 256" is away from the second guide wall 251" along the axial direction of the assembling part 112". In the positive direction, the first positioning block 255'" is located above the third positioning block 257"', and the second positioning block 256'" is located above the third positioning block 257" in the positive direction. Preferably, in this embodiment, the first positioning block 255", the second positioning block 256" and the third positioning block 257" are separately provided on two adjacently arranged elastic arms 253" with different heights. In order to ensure the flexibility of the elastic arm 253" to hold and abut.

The assembling part 112" is provided with a through hole 114" for receiving the elastic arm 253", and the through hole wall 115" of the side of the through hole 114" close to the elastic arm 253" is inclinedly arranged, when the elastic arm 253" moves toward the first trigger mechanism 24" along the axial direction of the assembly part 112", the through hole wall 115" abuts the third inclined guide surface 258" to push the elastic arm 253" along the assembly axial rotation of the portion 112".

In this embodiment, the elastic arm 253"' can slide from the initial position to the locked position via the ready position in the assembly part 112"'. Specifically, when the elastic arm 253" is in the initial position, the third positioning block 257" is clamped on the assembling part 112". At this time, the elastic arm 253" partially penetrates the assembling part 112" and is accommodated between the case 21" and the fixing part 111", the tip part/all of the injection needle 12" is contained in the elastic arm 253". This arrangement can effectively prevent the injection needle 12" from being used before use. The external environment is contaminated; on the other hand, the axial position of the injection needle 12" is fixed to prevent the injection needle 12" from being deflected or bent when the injection device (not shown) is connected.

Further, during the installation process of the injection device, the elastic arm 253" will slide along the axial direction of the assembling part 112" towards the top end of the fixing part 111" under the action of external force. During the sliding process, the through hole wall 115'" presses against the third inclined guide surface 258", pushes the elastic arm 253" to rotate in the axial direction of the assembling part 112" until the elastic arm 253" completely penetrates the through hole 114", and reaches the second trigger mechanism 25". In the ready position, at this time, the elastic element 23" is compressed to abut against the second resisting wall 252", and the proximal end of the injection needle 12" penetrates the elastic arm 253" and is inserted into the injection device.

After the drug injection is finished, when the injection device is removed, the elastic element 23'" drives the elastic arm 253" to slide along the axial direction of the needle seat 11" toward the side away from the fixing portion 111" under its own elastic force. Stop until it slides to the locked position. When the elastic arm 253" is in the locked position, the first positioning block 255" is clamped in the first groove 116", and the third positioning block 257" is received in the assembly. The second positioning block 256" enters into the assembly section 112" under the action of the elastic arm 253", and is held against the inner side of the top wall of the assembly section 112", and the injection needle 12". The top part/all of it is contained in the elastic arm 253". Preferably, in this embodiment, when the elastic arm 253" is in the locked position, the injection needle 12" is all contained in the elastic arm 253" to prevent the injection needle 12" from causing injury to the patient after use.

In summary, the safety injector needles 100, 100', 100" of the present invention are connected to the first trigger mechanism 24, 24', 24" and the case 21, 21', 21", and the second trigger mechanism The connection forms of 25, 25', 25" and the needle seat 12, 12', 12" are set so that the first trigger mechanism 24, 24', 24" and the second trigger mechanism 25, 25', 25" can slide along the extension direction of the case 21, 21', 21" under the action of the elastic elements 23, 23', 23", so as to inject the safety injector needle 100, 100', 100" after use The distal ends of the needles 12, 12', 12" are all received between the first trigger mechanism 24, 24', 24" and/or the needle seats 11, 11', 11", and the proximal end is at least partially contained in the first trigger mechanism 24, 24', 24", between the two trigger mechanisms 25, 25', 25" and/or the needle seats 11, 11', 11", the safety of the safety injector needle 100, 100', 100" of the present invention is ensured.

At the same time, by setting the connection form and relative position of the sliders 244, 244', 244" and the chutes 211, 211', 211", at least part of the elastic elements 23, 23', 23" will pass the first accommodating parts 241, 241', 241" of a trigger mechanism 24, 24', 24" are exposed to the outside of the case 21, 21', 21", so as to be compatible with unused safety injector needles 100, 100', 100" to distinguish between the safety injector needles 100, 100' and 100" of the present invention, while ensuring that the safety injector needles 100, 100', and 100" of the present invention have better protection functions, it also has the function of indication of the use status, which achieves the purpose of improving the practicability of the safety injector needles 100, 100', and 100".

Furthermore, the safety injector needle 100, 100', 100" of the present invention has a simple structure, which passes through the sliders 244, 244', 244", the chutes 211, 211', 211" and the elastic elements 23, 23', the 23" cooperation realizes the protection and indication function of the safety injector needle 100, 100', 100", reduces the production cost of the safety injector needle 100, 100', 100" of the present invention, and makes the safety injection of the present invention the needles 100, 100', 100" are suitable for production and use.

The above embodiments are only used to illustrate the technical solutions of the present invention and not to limit them. Although the present invention has been described in detail with reference to the preferred embodiments, those of ordinary skill in the art should understand that the technical solutions of the present invention can be modified or equivalently replaced. Without departing from the spirit and scope of the technical solution of the present invention.

## Claims

1. A safety injector needle, comprising a needle seat, an injection needle axially fixedly connected to the needle seat, and a trigger protection assembly assembled on the needle seat, wherein:
the needle seat includes an assembling part and a fixing part, and the assembling part is arranged in a hollow tube shape;
the trigger protection assembly includes a case, a trigger mechanism and an elastic element;
the case is arranged in a hollow tube shape and is fixedly connected to the assembling part, and the inner wall of the case is provided with a first displacement chute, a second displacement chute and a locking chute corresponding to the trigger mechanism;
the trigger mechanism includes a first trigger mechanism and a second trigger mechanism. The first trigger mechanism is provided with a first inclined guide surface, a limiting slider corresponding to the first displacement chute and the second displacement chute, and an elastic slider corresponding to the locking chute; the second trigger mechanism is provided with a second inclined guide surface for driving the first trigger mechanism to rotate in the axial direction of the assembling part;
under the action of external force, the first inclined guide surface and the second inclined guide surface cooperate with each other to drive the limiting slider and the elastic slider to rotate in the axial direction of the assembling part, so that the limiting slider turns from the first displacement chute to the second displacement chute, and when the limiting slider slides into the second displacement chute, the elastic slider is clamped in the locking chute inside.

2. The safety injector needle according to claim **1,** wherein the first displacement chute and the second displacement chute are spaced apart by a baffle, and the bottom of the baffle is provided with a partition is provided between the second displacement chute and the locking chute to limit the rotation direction and the rotation angle of the first trigger mechanism in the case.

3. The safety injector needle according to claim **2,** wherein the bottom of the locking chute is provided with a lug boss, and the elastic slider is clamped on the lug boss to define the first locking position of the trigger mechanism.

4. The safety injector needle according to claim **1,** wherein the first trigger mechanism comprises an extension portion and a receiving portion, and the extension portion protrudes from the case and partially receives the fixing part and/or the injection needle; the accommodating portion is formed by extending in the opposite direction from the extension portion, and is accommodated in the case, the accommodating portion includes a first guide wall, and the second trigger mechanism partially penetrates the assembling part is slidable along the axial direction of the assembling part.

5. The safety injector needle according to claim **4,** wherein the first inclined guide surface is provided on a side surface of the first guide wall extending in the axial direction of the assembling part, and the limiting slider and the elastic slider are arranged on the outer wall of the first guide wall.

6. The safety injector needle according to claim **4,** wherein the first guide wall is provided with at least two accommodating cavities for accommodating the elastic element, and a first resisting wall is provided in the accommodating cavity, and the elastic element resists between the first resisting wall and the second trigger mechanism to elastically connect the second trigger mechanism and the first trigger mechanism.

7. The safety injector needle according to claim **4,** wherein the second trigger mechanism further comprises a second guide wall corresponding to the first guide wall and a second guide wall spaced apart from the second guide wall. The elastic arm, and the second guide wall and the elastic arm both penetrate the assembling part.

8. The safety injector needle according to claim **7,** wherein the second inclined guide surface is provided on a side surface of the second guide wall extending in the axial direction of the assembling part, and is connected with the first inclined guide surface. Corresponding to an inclined guide surface, the assembling part is provided with a through hole for accommodating the second guide wall and the elastic arm, and the through hole wall of the through hole close to the second inclined guide surface is inclinedly arranged, when the second trigger mechanism moves toward the first trigger mechanism along the axial direction of the assembling part, the through hole wall abuts on the second inclined guide surface to push the second trigger mechanism to rotate along the axial direction of the assembling part.

9. The safety injector needle according to claim **7,** wherein the second guide wall is provided with a first positioning block, and the elastic arm is provided with a second positioning block and a third positioning block, which define the direction where the fixing part is located is a positive direction, the first positioning block is located above the third positioning block, and the second positioning block is located above the third positioning block.

10. The safety injector needle according to claim **9,** wherein the assembling part is provided with a first groove for accommodating the first positioning block/third positioning block and a first groove for accommodating the third positioning block. The second groove of the positioning block defines the direction in which the fixing part is located as a positive direction, the first groove is located above the second groove, and when the first positioning block is clamped in the first groove, when the third positioning block is clamped in the second groove, the second positioning block enters the assembling part and is held against the inner side of the top wall of the assembling part, and the injection needle is accommodated in the second trigger mechanism.
